# EUROPEAN PATENT APPLICATION

(11) **EP 2 177 225 A1**
(43) Date of publication of application: **21.04.2010**
(21) Application number: 07792545.1
(22) Date of filing: 15.08.2007
(51) Int. Cl.: A61K 31/7016, A61K 31/185, A61M 1/14, A61M 1/28, A61P 7/08

(54) **PERITONEAL DIALYSATE**

(71) Applicant: Cheiron Japan Co., Tokyo 156-0055 (JP)
(72) Inventor: YUGARI, Yasumi, Kamakura-shi Kanagawa 247-0053 (JP); SANAKA, Tsutomu, Tokyo 112-0002 (JP)
(74) Representative: Green, Mark Charles
(86) International application number: PCT/JP2007/065911
(87) International publication number: WO 2009/022417

(57) **Abstract**

This invention provides a safe and highly stable peritoneal dialysate, which causes neither peritoneal membrane disorders nor peritoneal sclerosis associated with frequently repeated peritoneal dialysis treatment, which can protect residual renal functions in chronic renal failure, which inhabits the progress of renal damage over a long period of time, and which enables peritoneal dialysis treatment of diabetic patients. This peritoneal dialysate contains 0.05 to 3.5 w/v% of taurine and 0.1 to 6.5 w/v% of trehalose as osmotic agents and has a pH value adjusted between 6.5-7.5.

## Description

### TECHNICAL FIELD

This invention relates to peritoneal dialysate used for peritoneal dialysis.

### BACKGROUND ART

Peritoneal dialysis is a therapeutic procedure in which waste products are drawn from bodily fluids across the peritoneum and into peritoneal dialysate by leaving, for a given length of time, peritoneal dialysate sufficiently injected into the abdominal cavity of a renoprival patient with acute or chronic kidney failure. It further has a purpose of regulating the balance of the components of the various body fluids through the medium of the therapeutic procedure. The peritoneal dialysis is generally carried out using a liquid solution referred to as peritoneal dialysate.

As one example of the liquid solutions, there is enumerated a dialysate solution for continuous ambulatory peritoneal dialysis (CAPD). There has been reported a dialysate solution which typically contains lactate salt as an alkaline agent and hydrogen carbonate in order for maintaining a pH value approximate to the body fluid, as well as electrolyte compositions such as sodium chloride, calcium chloride and magnesium chloride and further contains glucose as an osmotic agent for keeping the osmotic pressure higher than the body fluid in order to secure ultrapenneability of the dialysate solution (Patent Reference 1).

Patent Reference 1: Japanese Published Unexamined Application No. 2000-51348

### DISCLOSURE OF THE INVENTION

### PROBLEMS TO BE SOLVED BY THE INVENTION

However, peritoneal dialysate containing glucose, as described in Patent Reference 1, has a problem such that hyperglycemia attributable to glucose present at high concentration affects the whole body and metabolism. Particularly, since cases of kidney failure caused by diabetes have been remarkably increasing as of late, there is concern about the use of glucose for the peritoneal dialysate as it may aggravate kidney failure.

Further, when a glucose solution is adjusted to have a pH value of 6.0 to 7.5, with an aim to come into a neutral to alkaline state close to a physiologic condition, a subset of glucoses are polymerized with one another or react with residual trace substances when sterilized at a high temperature, consequently tingeing the glucose solution. Particularly when a small amount of amino acid is resident in the solution, a dramatic browning Maillard reaction occurs, and further, glucose decomposition products having peritoneal cytotoxicity are produced.

The use of glucose suffers from the disadvantage that glucose is decomposed at a neutral pH or a slightly alkaline pH to increase 5-hydroxymethylfurfiu-al (5-HMF), formic acid, aldehydes and others, consequently decreasing the pH value with time. These decomposition products have cytotoxic activity and may contribute to the development of complications such as amyloidosis and accelerated arteriosclerosis.

In order to suppress the aforementioned reaction, the pH value of the peritoneal dialysate is reduced to around 5.0, but a patient undergoing peritoneal dialysis at a low pH is constantly exposed to the acidic solution of highly concentrated glucose. Additionally, since glucose reacts, due to its carbonyl radical, with free amino acids of amino acid, peptides and proteins or phospholipids of the peritoneum, the peritoneum is damaged by long-term use of the highly concentrated glucose solution. This can lead to peritoneal deterioration and even peritoneal sclerosis.

To solve these problems, a glucose-replacing osmotic agent has been proposed. For instance, trehalose is widely distributed in the animal kingdom and is a stable disaccharide which is easily metabolized inside the body and also used to prevent adhesion at surgical sites. Patent Reference 2 discloses a peritoneal dialysate containing trehalose as an osmotic agent. Patent Reference 3 discloses a peritoneal dialysate containing trehalose as an osmotic agent and glucose as an energy source.

One of the inventors of this invention has disclosed a peritoneal dialysate in which taurine is selected as an osmotic agent (Patent Reference 4). Taurine is a non-protein amino acid and has been confirmed to oppose changes in osmotic pressure of the body fluid by increasing its intracellular concentration, thereby protecting cells. Taurine has further been confirmed by a load test to be considerably safe for humans. In addition, a peritoneal dialysis patient has a low taurine concentration in blood plasma and muscles due to synthesis inhibition, and thus it is effective to supply the taurine to the patient through the medium of the dialysate. Taurine may also have secondary effects of improving circulatory function and fat metabolism and diuretic activity, as well as being suitable for peritoneal dialysis.

Patent Reference 2: Japanese Published Unexamined Application HEI 07-323084
Patent Reference 3: Japanese Published Unexamined Application No. 2002-282354
Patent Reference 4: Japanese Published Unexamined Application No. 2005-531630

However, the peritoneal dialysate as described in Patent References 2-4 cannot be said to be a perfect replacement for peritoneal dialysate containing glucose, which has been the type of dialysate mainly used to date. The peritoneal dialysate described in Patent Reference 3 is typically used with a mixture of glucose, which has long been used, and trehalose, but problems potentially caused by the use of glucose, such as peritoneal injury and hyperglycemia, remain.

Although the peritoneal dialysate described in Patent References 2 and 4 is free of glucose, it has to contain an osmotic agent in relatively high concentration when only one osmotic agent is used. In that case, when the dialysate flows rapidly into the blood due to weakening of the peritoneum, which is possibly caused by frequent dialysis, the possibility of allowing high levels of agent to destabilize bodily metabolism is undeniable. In addition, it cannot be denied that when one substance assumes all the osmotic activities, the possibility of causing a change in pH and a reaction of contained material due to unexpected extracorporeal factors or intracorporeal factors during dialysis may pose a risk to the dialysis patient. To date, peritoneal dialysate containing osmotic agents other than glucose have not commonly been used, primarily because its biosafety for long-term use has not sufficiently been confirmed.

Peritoneal dialysate safe for use in a chronic renal failure patient is essentially required to have an appropriate osmolar design and characteristics for peritoneal dialysis, and function to sufficiently treat a renal failure patient without producing the various injurious effects caused by inclusion of glucose. Further, it must assure liberal safety. That is, it needs to be exceptionally stable so as to provide and maintain the optimal osmotic pressure and pH for ensuring safety in the body.

Such being the case, the inventors of this invention investigated a solution to the aforementioned problems by concomitant use of taurine and trehalose as osmotic agents. Taurine and h-ehalose can both safely maintain blood metabolic activity even when the blood concentration temporarily increase due to weakening of the peritoneum or other causes. At that time, if the concentration of each substance can be decreased without singly using one of the substances at high concentration, any impact on metabolism can be diminished so as to increase the safety. Both substances are equivalent as the osmotic agent and additively involved in the osmotic pressure, but they are metabolically different from each other, so that safety and stability can be improved synergistically by being kept at low concentration respectively.

In order to solve the above problems, the present invention seeks to provide peritoneal dialysate having little peritoneal cytotoxicity, being neutral (pH 6.5 to 7.5), having few side-effects and superior physicochemical stability, causing little biological perturbation, and being capable of satisfactorily attaining a blood purification effect for removing mea or urea-derived nitrogen-containing compounds to be treated and a dewatering effect by containing, as an osmotic agent, multiple innocuous natural ingredients other than glucose.

### MEANS FOR SOLVING THE PROBLEMS

To attain the object described above, the peritoneal dialysate according to the present invention comprises the following characteristic means:

That is, the peritoneal dialysate of the present invention is characterized by comprising 0.05 to 3.5 % w/v of taurine and 0.1 to 6.5 % w/v of trehalose and being pH 6.5 to 7.5.

Further, the peritoneal dialysate of the present invention is characterized by using taurine and trehalose as osmotic agents and having osmotic pressure of 300 to 680 mOsm/L based on extracellular fluid.

Further, the peritoneal dialysate of the present invention is characterized by comprising taurine, trehalose, amino acids, minerals, vitamins and an alkaline agent.

Further, the peritoneal dialysate of the present invention is characterized by containing an amino acid selected from L-histidine, L-isoleucine, L-leucine, L-valine, L-tyrosine, L-arginine, L-tryptophan, L-lysine, L-methionine, L-phenylalanine, L-threonine, L-glutamine, L-glutamic acid, and L-carnitine, and at least L-histidine, L-isoleucine, L-leucine, L-valine, L-tyrosine, and L-arginine, and having an amino-acid concentration of 0.4 to 5.0 % by weight in sum total.

Further, the peritoneal dialysate of the present invention is characterized by containing, as the minerals, three or more kinds selected from ferric iron, copper ion, calcium ion, magnesium ion, zinc ion, chromium ion, selenium ion, and manganese ion.

Further, the peritoneal dialysate of the present invention is characterized by containing, as the vitamins, at least four or more kinds selected from vitamin A, vitamin D3, vitamin E, vitamin B1, vitamin B2, niacin, vitamin B6 and its derivative, folic acid, vitamin B12, pantothenic acid, vitamin C and its derivative, wherein the total amount of the vitamins is 10 to 40 mg.

Further, the peritoneal dialysate of the present invention is characterized by containing bicarbonate as the alkaline agent, wherein the pH value thereof is adjusted to 6.5 to 7.5 in use.

### EFFECT OF THE INVENTION

The present invention formulated as above brings about the following effects.

According to the present invention, the peritoneal dialysate suitable for use in peritoneal dialysis can be obtained by mixing both osmotic agents while adjusting the ratio of concentration in the range of 0.05 to 3.5 % w/v of taurine and 0.1 to 6.5 % w/v of trehalose, so that the osmotic pressure can be adjusted arbitrarily within a range capable of ensuring a dewatering amount sufficient for dialysis to an extent of causing no water loss. The peritoneal dialysate obtained by mixing the osmotic agents at the aforementioned ratio of concentration can synergistically protect and preserve the peritoneal tissue by the action of protecting and repairing tissue and cells by distinctive functions of the osmotic agents while providing an osmotic pressure effect by virtue of each of the substances contained therein, consequently preventing development of a disorder. More stable and safe osmotic pressure can be retained against unexpected disorders caused by relying on only one osmotic agent.

The peritoneal dialysate according to the present invention does not require the addition of glucose as an osmotic agent, thus removing any need to make the pH value acidic in order to prevent undesirable chemical reactions of glucose. This allows for a pH value of 6.5 to 7.5, which is close to physiological conditions, thereby obviating the risk to the dialysis patient of any shock brought on by an acidic solution. Further, this avoids disorders arising from chemical reactants of glucose and elevation of blood sugar level, so that the peritoneal dialysis can be carried out more safely over a prolonged period.

The peritoneal dialysate according to the present invention can provide safety in excess of a established ranges of conventional dialysis by establishing the minimum limit value of osmotic pressure at 300 mOsm/L. The maximum limit value of osmotic pressure is set to 680 mOsm/L, which does not cause rapid dewatering, so that an optimum dewatering speed for performing peritoneal dialysis can be achieved.

The peritoneal dialysate according to the present invention contains all or part of amino acids, minerals and vitamins, so that furnishing of nutrition can be performed through dialysis, consequently protecting a patient from nutritional deficiency and defective metabolism caused by dialysis. Particularly, since glucose is not used as an osmotic agent, there is no need to worry about the carbonyl group of glucose reacting with various amino acids, peptides and other proteins (various types of membrane proteins such as hemocyte membrane). Furthermore, the osmotic agent does not mutually react with these added nutrients by virtue of the pH stability brought about by the alkaline agent, so that it can be maintained stably.

According to the peritoneal dialysate of the present invention, amino acids can be added for nutritional support because the osmotic agents are both unresponsive to amino acids. The profile of amino acids used to supplement the peritoneal dialysate of the invention has the significance of not merely adding amino acids, centered around the essential amino acids, for the purpose of conserving and fortifying protein nutrition, but also coping with the imbalance of amino-acid metabolism resulting from organ dysfunction such as renal failure. Leucine, isoleucine and valine, which are branched-chain amino acids and constituent elements of muscle, serve to alleviate muscle depletion caused by a low protein diet in patients with renal failure. Histidine is synthesized into muscle protein and it is one of blood pigment components, so that it can be suitably prescribed for an anemic condition. Arginine, which is reduced in synthesis due to renal failure, is important for maintaining immunity activity and indispensable for nitrogen metabolism and has a vasodepressor effect and an insulin-secretion stimulating effect. Tyrosine, as a precursor of a neurotransmitter, serves to supplement the activities of the nervous system. By adding a suitable quantity of amino acids to the peritoneal dialysate, aggressive therapy can be provided for improving metabolism and aiming to maintain and preserve nephron function by using the same peritoneal dialysate, thus defining new dimensions in dialysis therapy for renal failure. Hence, there is no precedent for amino-acid addition to the peritoneal dialysate for furnishing nutrition and fulfilling a specific function for therapeutic applications.

According to the peritoneal dialysate of the present invention, essential trace minerals such as copper, zinc, manganese, chromium and selenium, in addition to calcium, magnesium and iron, can be added to the peritoneal dialysate. The minerals are indiscriminately excreted and drawn into the dialysate during peritoneal dialysis therapy, potentially leading to an increased burden on a dialysis patient due to alimentary deficiency and enhanced general fatigue. The ferric ion and copper ion as disclosed in this invention are added for coping with fenic-ion or copper-ion deficiency anemia, and the calcium ion is added for its protective effect against osteoporosis. The magnesium ion is added for biomembrane conservation and normalization of energetic metabolism or muscular movement, the zinc ion is added for preventing outbreak of dennatitis and conserving immunity, and the chromium ion is added for preventing peripheral nerve impairment and decreased carbohydrate tolerance. The selenium has a function of suppressing development of cardiomyopathy and leg myalgia. There has not yet been such a peritoneal dialysate containing these essential trace minerals.

The peritoneal dialysate of the invention can contain vitamins because glucose is excluded, which allows for making the pH value neutral. Thus, the dialysis patient can be protected from vitamin deficiency resulting from diet restriction. Since it is specifically possible to add a vitamin B6 derivative, it should be feasible to eliminate glucose-derived harmful substances such as GDP (glucose degradation products) and AGE (advanced glycation end-products), thereby potentially reducing the risk of peritoneal sclerosis. The addition of vitamins to the conventional peritoneal dialysate has never been taken into consideration.

The peritoneal dialysate of the invention can be stably kept at a neutral pH value close to physiological conditions by the use of bicarbonate as the alkaline agent. The constituents of the peritoneal dialysate of the present invention set forth in claims 1 to 6 can eliminate the need for lowering the pH value to ensure stability when dissolving the dialysate, so that the pH value can be adjusted within a neutral range, thereby making the dialysate stable. Though there is little possibility of dramatically departing from the range of physiological conditions, the fact that the peritoneal dialysate of the invention already contains the alkaline buffering agent means that the pH value can be kept within a safer range should any unanticipated situations arise during use. Father, by selecting as the alkaline agent bicarbonate, which serves in vivo as a pH buffering agent for blood, the dialysate more closely resembles the internal environment of the living organism.

### BEST MODE FOR CARRYING OUT THE INVENTION

The present invention will be described hereinafter in detail.

The trehalose subsumes, as its isomer, α,β-trehalose and β,β-trehalose. As the osmotic agent, the natural α,α-trehalose is used in this invention. Taurine and trehalose do not react mutually with each other, so these osmotic agents can be mixed at an arbitrary concentration and at an arbitrary ratio of concentration designed for the peritoneal dialysate. Because the aforementioned trehalose is a disaccharide, its use as an osmotic agent must be at double the concentration of a monosaccharide, such as glucose, in order to possess equivalent osmotic activity.

The contained amount of the osmotic agents is 0.05 to 3.5 % w/v of taurine and 0.1 to 6.5 % w/v of trehalose. In case of a concentration higher than this, the dewatering amount becomes excessive, which can lead to various symptoms associated with water loss. The excess dewatering may potentially cause peritoneal sclerosis due to chronic osmotic stress. To determine a concentration at which osmotic pressure produces a satisfactory dialyzing effect, and at which dewatering is not caused excessively, it is preferable to specify a range of 0.1 to 3.0 % w/v of taurine and 0.2 to 6.0 % w/v of trehalose, more preferably, 0.3 to 2.5 % w/v of taurine and 0.6 to 5.0% w/v of trehalose, for obtaining an effectual dewatering amount.

The osmotic pressure of the peritoneal dialysate is adjusted by substantially controlling the concentration of the osmotic agents, i.e. taurine and trehalose, or adjusting the ratio of concentration of both osmotic agents. Upon withholding the contribution of all other dissolved substances to the osmotic pressure, the osmotic pressure of the dialysate can be defined by taurine and trehalose as the osmotic agents, but the mixing ratio (molar ratio of concentration) of both substances may be determined to 10:1 1 to 1:10. The concentration of both substances may more preferably be determined to be kept close to an equal mole ratio of concentration.

The osmotic pressure defined by the addition of all components is required to be in a range of 300 to 680 mOsm/L. When the osmotic pressure is less than 300 mOsm/L, the dewatering amount hardly reaches the required amount. When a mixture of the osmotic agents having a concentration of no less than 680 mOsm/L is used, the dewatering amount becomes excessive, which can lead to the development of various symptoms associated with water loss. It is more preferable to make the osmotic pressure between 320 mOsm/L and 650 mOsm/L, so as to provide a sufficient dewatering amount, thereby avoiding rapid dewatering, which acutely causes physiological impact, and preventing peritoneal sclerosis due to chronic osmotic stress. The osmotic pressure in the range of 330 to 530 mOsm/L is particularly advisable for carrying out gradual dewatering.

The amino acids to be added to the dialysate are selected from L-histidine, L-isoleucine, L-leucine, L-valine, L-tyrosine, L-arginine, L-tryptophan, L-lysine, L-methionine, L-phenylalanine, L-threonine, L-glutamine, L-glutamic acid, L-proline and L-carnitine. The particularly desirable concentration of the amino acids may be achieved by using six or more components of 0.02 to 0.13 % w/v of L-histidine, 0.085 to 0.50 % w/v of L-isoleucine, 0.07 to 0.8 % w/v of L-leucine, 0.05 to 0.5 % w/v of L-valine, 0.02 to 0.15 % w/v of L-tyrosine, 0.025 to 0.6 % w/v of L-arginine, 0.02 to 0.20 % w/v of L-tryptophan, 0.02 to 0.3 % w/v of L-methionine, and 0.05 to 0.5 % w/v of L-carnitine. The concentration of each amino acid shows a desirable concentration for restoring the aminogram of a patient undergoing dialysis therapy to the normal range. If the concentration is excessive, the increased risk of causing acidosis requires attention. A variety of investigations have found that the concentration of the amino acid to be added should be 0.4 to 5.0 % w/v in terms of the total amount of amino acid, more preferably, 0.7 to 3.0 % w/v for lessening the risk of metabolic acidosis.

The dialysate for a one-time use preferably contains, as the minerals, ferric iron (0.1 to 3 mg), copper ion (0.1 to 0.7 mg), chromium ion (0.1 to 50 µg), manganese ion (0.1 to 0.5 mg), selenium ion (0.1 to 20 µg), calcium ion (1.0 to 10.0 mg), magnesium ion (0.2 to 4.0 mg), and zinc ion (0.2 to 2.0 mg). Although the water-soluble essential trace minerals in the blood are lost due to the peritoneal dialysis, the amount of metals to be replenished as additives to the dialysate conforms to 'Nutritional Requirements of Japanese (Sixth Edition)'.

Vitamins should be offered for compensating for components lost with dewatering during peritoneal dialysis therapy. In particular, vitamin B6 derivatives are useful for enhancing an antioxidant effect. As to the types and the additive amounts of the respective vitamins to be added per one dose of dialysate, the dialysate may preferably contain six or more of vitamin A (100 to 2000 I.U.), vitamin D3 (10 to 200 I.U.), vitamin E (0.5 to 10mg), vitamin B1 (0.2 to 5.0mg), vitamin B2 (0.5 to 5mg), niacin (2 to 10mg), vitamin B6 and one or two or more derivatives thereof, i.e. pyridoxine, pyridoxal, pyridoxamine, and pyridoxal phosphate, (0.2 to 10mg, respectively), folate (50 to 200µg), vitamin B12 (1.0 to 5.0µg), pantothenic acid (0.5 to 5mg), and vitamin C or ascorbic acid 2-glucoside (20 to 100mg). The amounts defined herein conform to those described in the 'Nutritional Requirements of Japanese (Sixth Edition)' and suffice to replenish the amounts and qualities of the components lost during peritoneal dialysis.

The pH value of the peritoneal dialysate is determined and maintained in the range of 6.5 to 7.5. Although the present invention employs, as the alkaline agent, hydrogen carbonate, which serves as a blood-buffering agent in vivo, lactate salt and citric salt may be used as well. Electrolytes generally used in peritoneal dialysis include, e.g., sodium ion, calcium ion, magnesium ion, zinc ion, and chlorine ion. Electrolyte compositions containing sodium chloride, calcium chloride, magnesium chloride and zinc sulfate are preferable. Taurine and trehalose as osmotic agents can be maintained stably in the above-mentioned pH range, consequently avoiding reactions with other components of the dialysate, interperitoneal compositions or peritoneal cells.

The compounding amount of each of the components contained in the peritoneal dialysate may preferably be determined in the concentration range as enumerated below.

| | |
|---|---|
| Sodium ion | 50 to 150 mEq/L |
| Potassium ion | 0 to 3.0 mEq/L |
| Chlorine ion | 50 to 140 mEq/L |
| Alkaline agent | 2.0 to 45 mEq/L |
| Total of trehalose and taurine | 0.6 to 9.0 % w/v |
| Total of amino acids | 0.4 to 5.0 % w/v |

The peritoneal dialysate is stored in a plastic contained made of e.g., polyethylene, polypropylene, polyvinyl chloride, polyester, ethylene-vinyl acetate copolymer, nylon, or complex material thereof. The present invention containing a mixture of trehalose and taurine as the osmotic agents in place of glucose may be stored in a container having a singe compartment as a neutral dialysate without peritoneal cytotoxicity. However, if the need arises, the dialysate may also be formulated for storage in containers with two or more compartments.

Sterilization may be performed under ordinary heat sterilization conditions at 108 degrees C for 60 minutes, but use of a temperature-resistant container enables 'all kill' sterilization. Of coarse, other sterilization methods may be applied, e.g., aseptic filtration sterilization and radiosterilization using ultraviolet ray or gamma ray. The invention can be put to practical use in such a manner that CAPD dialysate is put in a plastic container having no gas-banier properly and covered with a gas-bamer packing material on the outside, or it can be stored in a gas-barrier plastic container. The term 'material having gas-barrier property' means a material having non-permeability or little permeability to gases such as oxygen, nitrogen, carbon dioxide and water vapor. The term 'gas-barrier plastic' means, for instance, ethylene-vinyl alcohol copolymer, polyvinylidene chloride, gas-barrier nylon or other polymers. The optimal gas-barrier plastic can be selected from these materials so as to gain an appropriate degree of polymerization. The gas-barrier plastic may be made by laminating or coating a plastic base with these materials, or by laminating or coating with aluminium, aluminum oxide, silicon oxide or the like. Transparency and opacity thereof do not matter.

In case a gas-banier packing material is used as an outer packing material, a space between the container storing the peritoneal dialysate and the outer packing material may be filled with an inert gas, or a moderately concentrated mixture of inert gases, such as nitrogen gas and carbon dioxide or sealed in a de-aerated state.

### EMBODIMENT

Hereinafter, preferred embodiments of the invention will further be described in detail, but the invention is not to be considered limited to these embodiments.

### EMBODIMENT 1: (stability with respect to heat sterilization)

A solution was made by dissolving, into a suitable quantity of distilled water for injection, 107.8g of sodium chloride, 5.14g of calcium chloride dihydrate, 1.016g of magnesium chloride hexahydrate, 5.4g of acid sodium carbonate, 5.6g of L-histidine, 3.8g of L-tryptophan, 15.0g of L-isoleucine, 12.4g of L-leucine, 9.4g of L-valine, 4.3g of L-tyrosine, and 9.4g ofL-arginine, and adjusting the pH value to 7 with sodium hydrate, followed by adding water up to 20 liters in volume. The solution thus obtained was designated as a basic solution. The basic solution in this embodiment does not contain vitamins. Test solutions TT-1, TT-2, TT-3, TT-4 and Gluc. are shown in the following Table 1.

**TABLE 1**

| Test Solutions | Measuring Time | Measurement Item | | | Aspect |
|---|---|---|---|---|---|
| | | pH | Absorbance (430 nm) | Osmotic Pressure | |
| Basic Solution | Before sterilization | 7.05 | 0.005 | 309 | Transparent |
| | After sterilization | 6.85 | 0.000 | 312 | Transparent |
| TT-1 | Before sterilization | 7.21 | 0.009 | 405 | Transparent |
| | After sterilization | 6.93 | 0.019 | 407 | Transparent |
| TT-2 | Before sterilization | 7.01 | 0.002 | 458 | Transparent |
| | After sterilization | 6.85 | 0.008 | 460 | Transparent |
| TT-3 | Before sterilization | 6.98 | 0.003 | 521 | Transparent |
| | After sterilization | 6.72 | 0.024 | 524 | Transparent |
| TT-4 | Before sterilization | 7.15 | 0.000 | 395 | Transparent |
| | After sterilization | 6.95 | 0.011 | 394 | Transparent |
| Comparison Solution Gluc. | Before sterilization | 7.65 | 0.000 | 512 | Transparent |
| | After sterilization | 7.55 | 0.126 | 516 | Colored (Yellow) |

| | | | | | |
|---|---|---|---|---|---|
| Basic solution: 20 liters of distilled water for injection, containing only salts and amino acids and having pH 7.4. TT-1: Basic solution (1L) + Taurine (10.0g) + Trehalose (10.0g) TT-2: Basic solution (1L) + Taurine (15.0g) + Trehalose (15.0g) TT-3: Basic solution (1L) + Taurine (30.0g) TT-4: Basic solution (1L) + Trehalose (30.0g) Gluc.: Basic solution (1L) + Glucose (38.0g) Sterilization Condition: 108 degrees C for 60 minutes Preservation Condition: Left at 60 degrees C | | | | | |

As is evident from the results shown in Table 1, the test solutions TT-1 to TT-4 changed little in aspect, pH value and osmotic pressure even after high-pressure steam sterilization and storage at 60 degrees C for two weeks, and the stability of the test solutions could be maintained. On the other hand, a comparison solution containing glucose underwent a severe browning reaction after high-pressure steam sterilization, thereby leading to a lower pH value. After storing for two weeks, this glucose-containing solution developed a tendency to brown further and decrease the pH value yet more.

### EMBODIMENT 2: (Relation between concentration and dewatering amount)

A solution was regulated in quantity by dissolving, into distilled water, sodium chloride (5.38g), sodium hydrogen carbonate (2.52g), magnesium chloride hexahydrate (34mg), calcium chloride dihydrate (370mg), and zinc sulfate heptahydrate (86mg), so as to have a pH value of 7.2, and adding distilled water up to 1 liter of the regulated solution. The obtained solution was designated as a basic solution. To the basic solution, 2.0g of L-arginine, 0.5g of L-histidine, 3.0g of L-leucine, 2.0g of L-isoleucine, 2.0g of L-valine and 0.5g of L-tyrosine were added, and further, taurine and trehalose were added to obtain test solutions TTA-3 to TTA-6. Comparison solutions GLU-1, GLU-2 and GLU-3 were prepared by dissolving glucose into the basic solution. The solutions TTA-1 and TTA-2 each had an amino-acid concentration half of that of the respective TTA-3 to 6. Upon injecting the solutions each were injected into the abdominal cavity of a male SD rat, the fluid volume of each solution injected into the abdominal cavity was measured to determine a dewatering amount based on a difference between the measured volume and the injected volume (30 ml).

The results are shown in Table 2.

**TABLE 2**

| | Osmotic Agent (g/L) | | | | Average Dewatering Amount (ml) | | | Osmotic Pressure |
|---|---|---|---|---|---|---|---|---|
| | Amino Acid | Taurine | Trehalose | Glucose | Average | Minimum | Maximum | |
| Basic Solution | 0 | 0 | 0 | 0 | -5.16 | -5.31 | -5.03 | 251.9 |
| TTA-1 | 5 | 0.03 | 0.06 | 0 | -2.43 | -3.03 | -2.1 | 288.7 |
| TTA-2 | 5 | 0.50 | 1.00 | 0 | -1.84 | -1.89 | -1.53 | 295.1 |
| TTA-3 | 10 | 3.75 | 4.25 | 0 | 0.58 | 0.19 | 0.76 | 330.0 |
| TTA-4 | 10 | 3.75 | 10.80 | 0 | 4.23 | 3.74 | 4.53 | 384.7 |
| TTA-5 | 10 | 5.62 | 5.40 | 0 | 4.57 | 4.33 | 4.75 | 385.3 |
| TTA-6 | 10 | 7.50 | 10.80 | 0 | 6.71 | 6.47 | 6.90 | 414.7 |
| TTA-7 | 10 | 10.00 | 14.40 | 0 | 8.57 | 8.32 | 9.25 | 446.6 |
| GLU-1 | 0 | 0 | 0 | 13.6 | 0.61 | -0.11 | 0.30 | 327.5 |
| GLU-2 | 0 | 0 | 0 | 22.7 | 4.26 | 3.80 | 4.63 | 378.1 |
| GLU-3 | 0 | 0 | 0 | 38.6 | 11.54 | 11.29 | 11.79 | 466.4 |

As shown in Table 2, the peritoneal dialysate containing the osmotic agent prepared by mixing taurine, trehalose, and six types of amino acids can bring about concentration-independent osmotic pressure, as well as a solution containing glucose as the osmotic agent, so that a desirable dewatering effect can be achieved by control of concentration.

### Embodiment 3: (Peritoneal cytotoxicity)

It is a basic requirement for the peritoneal dialysate that the peritoneum is maintainable in its dewatering function over a lengthy period without being harmed by repeated injection, dwelling and drainage of the peritoneal dialysate relative to the peritoneum. For a comparative investigation of peritoneal cytotoxicity, the following experimental conditions are provided. To be specific, a given volume (30 ml) of testing peritoneal dialysate was injected into the abdominal cavity of a rat, and subsequently drained following a dwelling time of a given length (four hours). Subsequently, the same volume of other identical peritoneal dialysate was injected into the abdominal cavity and all drained following a dwelling time of a given length (four hours). This procedure was performed three times a day and continued for seven days. When the peritoneum is damaged, the dewatering competence is reduced according to extent of damage, thus, the peritoneal cytotoxicity of the testing peritoneal dialysate can be evaluated by injecting an assay reagent (30 ml) containing xylitol having a fixed concentration into the abdominal cavity on the eighth day after start of the test, and then draining the solution after the elapse of a given dwelling time (four hours), after which the volume of the drained solution and the concentration of xylitol can be measured. For use as the testing peritoneal dialysate, the peritoneal dialysate TTA-3 according to the invention (cf. Embodiment 2) was used, and as the comparison solution, a commercially available peritoneal dialysate containing 2.27 % of glucose was used. Using five male SD rats (with weight of 250 up to 300 g), injection and drainage of 30 ml of the testing peritoneal dialysate and the same quantity of comparison solution were repeatedly performed three times a day for seven days, and on the eighth day after start of the test, 30 ml of 1.9 % w/v testing xylitol solution (special grade xylitol made by Wako Pure Chemical Industries, Ltd.) was injected into the abdominal cavity of the rats. Just after injection, a specimen was collected and, after being left for four hours, again collected in order to measure the concentration of xylitol, and then all of the solution was drained to measure the volume of the discharged solution. Assuming that the volume of xylitol just after injection is D0 and the volume of the same after being left for four hours is D4, profuse dispersal of xylitol outside the peritoneum, as shown by the ratio of D4/D0 decreasing, indicates the possibility of disease or disorder of the peritoneum.

**TABLE 3: Quantitative Ratio D4/D0 (N=5)**

| Testing Peritoneal Dialysate | | |
|---|---|---|
| Xylitol Volume (g) | TTA-3 | 2.27 % Glucose-Containing Commercial Peritoneal Dialysate |
| D0 | 0.564±0.012 | 0.564±0.014 |
| D4 | 0.133±0.012 | 0.080±0.015 |
| D4/D0 | 0.235±0.024 | 0.142±0.022 |

As shown in Table 3, the xylitol quantitative ratio D4/D0 relative to TTA-3 according to the invention significantly indicates an elevated level compared with the commercially available peritoneal dialysate (containing 2.27 % of glucose) (P<0.001). That is, even with frequent, repetitive injection and drainage of the peritoneal dialysate TTA-3 relative to the abdominal cavity, the amount of peritoneal dialysate outflowing from the abdominal cavity into the blood vessel through the peritoneum is significantly smaller than that in the case of using the glucose-containing peritoneal dialysate, indicating little, if any, disease or disorder of the peritoneum.

### INDUSTRIAL APPLICABILITY

The peritoneal dialysate according to the invention has superior physicochemical stability and causes little biological perturbation, does not involve peritoneal degradation, has fewer side-effects and, further, can attain a satisfactorily therapeutic blood purification effect for removing urea or urea-derived nitrogen-containing compounds and a dewatering effect, so that dialysis treatment can safely be provided for a renal failure patient for long periods.

## Claims

1. Peritoneal dialysate comprising 0.05 to 3.5 % w/v of taurine and 0.1 to 6.5 % w/v of trehalose and being pH 6.5 to 7.5.

2. Peritoneal dialysate comprising famine and trehalose as osmotic agents and having osmotic pressure of 300 to 680 mOsm/L based on extracellular fluid.

3. The peritoneal dialysate set forth in claim 1 or claim 2, further containing amino acids, minerals and vitamins.

4. The peritoneal dialysate set forth in claim 1 or claim 2, further containing amino acids selected from L-histidine, L-isoleucine, L-leucine, L-valine, L-tyrosine, L-arginine, L-tryptophan, L-lysine, L-methionine, L-phenylalanine, L-threonine, L-glutamine, L-glutamic acid, L-proline and L-canutine, and at least L-histidine, L-isoleucine, L-leucine, L-valine, L-tyrosine, and L-arginine, and having an amino-acid concentration of 0.4 to 5.0 % w/v by weight in sum total.

5. The peritoneal dialysate set forth in claim 3, further containing amino acids selected from L-histidine, L-isoleucine, L-leucine, L-valine, L-tyrosine. L-arginine, L-tryptophan, L-lysine, L-methionine, L-phenylalanine, L-threonine, L-glutamine, L-glutamic acid, L-proluie and L-camitine, and at least L-histidine, L-isoleucine, L-leucine, L-valine, L-tyrosine, and L-arginine, and having an amino-acid concentration, of 0.4 to 5.0 % w/v by weight in sum total.

6. The peritoneal dialysate set forth in claim 1 or claim 2, further containing, as the minerals, three or more kinds selected from ferric iron, copper ion, calcium ion, magnesium ion, zinc ion, chromium ion, selenium ion, and manganese ion.

7. The peritoneal dialysate set forth in claim 3, further containing, as the minerals, three or more kinds selected from ferric iron, copper ion, calcium ion, magnesium ion, zinc ion, chromium ion, selenium ion, and manganese ion.

8. The peritoneal dialysate set forth in claim 4, further containing, as the minerals, three or more kinds selected from ferric iron, copper ion, calcium ion, magnesium ion, zinc ion, chromium ion, selenium ion, and manganese ion.

9. The peritoneal dialysate set forth in claim 5, further containing, as the minerals, three or more kinds selected from ferric iron, copper ion, calcium ion, magnesium ion, zinc ion, chromium ion, selenium ion, and manganese ion.

10. The peritoneal dialysate set forth in claim 1 or claim 2, further containing, as the vitamins, at least four or more kinds selected from vitamin A, vitamin D3, vitamin E, vitamin B1, vitamin B2, niacin, vitamin B6 and its derivative, folic acid, vitamin B 12, pantothenic acid, vitamin C and its derivative, wherein the total amount of the vitamins is 10 to 40 mg.

11. The peritoneal dialysate set forth in claim 3, further containing, as the vitamins, at least four or more kinds selected from vitamin A, vitamin D3, vitamin E, vitamin B1, vitamin B2, niacin, vitamin B6 and its derivative, folic acid, vitamin B12, pantothenic acid, vitamin C and its derivative, wherein the total amount of the vitamins is 10 to 40 mg.

12. The peritoneal dialysate set forth in claim 4, further containing, as the vitamins, at least four or more kinds selected from vitamin A, vitamin D3, vitamin E, vitamin B1, vitamin B2, niacin, vitamin B6 and its derivative, folic acid, vitamin B12, pantothenic acid, vitamin C and its derivative, wherein the total amount of the vitamins is 10 to 40 mg.

13. The peritoneal dialysate set forth in claim 5, further containing, as the vitamins, at least four or more kinds selected from vitamin A, vitamin D3, vitamin E, vitamin B1, vitamin B2, niacin, vitamin B6 and its derivative, folic acid, vitamin B12, pantothenic acid, vitamin C and its derivative, wherein the total amount of the vitamins is 10 to 40 mg.

14. The peritoneal dialysate set forth in claim 6, further containing, as the vitamins, at least four or more kinds selected from vitamin A, vitamin D3, vitamin E, vitamin B1, vitamin B2, niacin, vitamin B6 and its derivative, folic acid, vitamin B12, pantothenic acid, vitamin C and its derivative, wherein the total amount of the vitamins is 10 to 40 mg.

15. The peritoneal dialysate set forth in claim 7, further containing, as the vitamins, at least four or more kinds selected from vitamin A, vitamin D3, vitamin E, vitamin B1, vitamin B2, niacin, vitamin B6 and its derivative, folic acid, vitamin B12, pantothenc acid, vitamin C and its derivative, wherein the total amount of the vitamins is 10 to 40 mg.

16. The peritoneal dialysate set forth in claim 8, further containing, as the vitamins, at least four or more kinds selected from vitamin A, vitamin D3, vitamin E, vitamin B1, vitamin B2, niacin, vitamin B6 and its derivative, folic acid, vitamin B 12, pantothenic acid, vitamin C and its derivative, wherein the total amount of the vitamins is 10 to 40 mg.

17. The peritoneal dialysate set forth in claim 9, further containing, as the vitamins, at least four or more kinds selected from vitamin A, vitamin D3, vitamin E, vitamin B1, vitamin B2, niacin, vitamin B6 and its derivative, folic acid, vitamin B 12, pantothenic acid, vitamin C and its derivative, wherein the total amount of the vitamins is 10 to 40 mg.

18. The peritoneal dialysate set forth in claim 1 or claim 2, further containing, bicarbonate as the alkaline agent, wherein the pH value thereof is adjusted to 6.5 to 7.5 in use.

19. The peritoneal dialysate set forth in claim 3, further containing bicarbonate as the alkaline agent, wherein the pH value thereof is adjusted to 6.5 to 7.5 in use.

20. The peritoneal dialysate set forth in claim 4, further containing, bicarbonate as the alkaline agent, wherein the pH value thereof is adjusted to 6.5 to 7.5 in use.

21. The peritoneal dialysate set forth in claim 5, further containing bicarbonate as the alkaline agent, wherein the pH value thereof is adjusted to 6.5 to 7.5 in use.

22. The peritoneal dialysate set forth in claim 6, further containing bicarbonate as the alkaline agent, wherein the pH value thereof is adjusted to 6.5 to 7.5 in use.

23. The peritoneal dialysate set forth in claim 7, further containing bicarbonate as the alkaline agent, wherein the pH value thereof is adjusted to 6.5 to 7.5 in use.

24. The peritoneal dialysate set forth in claim 8, further containing bicarbonate as the alkaline agent, wherein the pH value thereof is adjusted to 6.5 to 7.5 in use.

25. The peritoneal dialysate set forth in claim 9, further containing bicarbonate as the alkaline agent, wherein the pH value thereof is adjusted to 6.5 to 7.5 in use.

26. The peritoneal dialysate set forth in claim 10, further containing bicarbonate as the alkaline agent, wherein the pH value thereof is adjusted to 6.5 to 7.5 in use.

27. The peritoneal dialysate set forth in claim 11, further containing bicarbonate as the alkaline agent, wherein the pH value thereof is adjusted to 6.5 to 7.5 in use.

28. The peritoneal dialysate set forth in claim 12, further containing bicarbonate as the alkaline agent, wherein the pH value thereof is adjusted to 6.5 to 7.5 in use.

29. The peritoneal dialysate set forth in claim 13, further containing bicarbonate as the alkaline agent, wherein the pH value thereof is adjusted to 6.5 to 7.5 in use.

30. The peritoneal dialysate set forth in claim 14, further containing bicarbonate as the alkaline agent, wherein the pH value thereof is adjusted to 6.5 to 7.5 in use.

31. The peritoneal dialysate set forth in claim 15, further containing bicarbonate as the alkaline agent, wherein the pH value thereof is adjusted to 6.5 to 7.5 in use.

32. The peritoneal dialysate set forth in claim 16, further containing bicarbonate as the alkaline agent, wherein the pH value thereof is adjusted to 6.5 to 7.5 in use.

33. The peritoneal dialysate set forth in claim 17, further containing bicarbonate as the alkaline agent, wherein the pH value thereof is adjusted to 6.5 to 7.5 in use.
